# EUROPEAN PATENT SPECIFICATION

(11) **EP 1 200 148 B1**
(45) Date of publication and mention of the grant of the patent: **05.11.2003**
(21) Application number: 00956311.5
(22) Date of filing: 27.07.2000
(51) Int. Cl.: A61M 15/00, B41M 5/26, B44C 1/00

(54) **MARKING METHOD**
MARKIERUNGSVERFAHREN
PROCEDE DE MARQUAGE

(30) Priority: 31.07.1999 GB 9917892
(43) Date of publication of application: 02.05.2002
(73) Proprietor: GLAXO GROUP LIMITED, Greenford, Middlesex UB6 ONN (GB)
(72) Inventor: GODFREY, James, William Glaxo Wellcome plc., Herts. SG12 0DP (GB); HAILEY, Mark, Andrew Glaxo Wellcome plc., Herts. SG12 0DP (GB); KOWALSKI, Julian, Zbigniew Glaxo Wellcome plc., Herts. SG12 0DP (GB)
(74) Representative: Rice, Jason Neale
(86) International application number: EP0007223
(87) International publication number: WO01008733

(56) References cited:
- EP-A- 0 190 997
- WO-A-98/56444
- US-A- 4 803 336
- J.L.C. WIJERS: "Industrieel lasergraveren baant zich een weg" POLYTECHNISCH TIJDSCHRIFT, WERKTUIGBOUW., vol. 46, no. 1, January 1991 (1991-01), pages 40-44, XP000233960 STAM TIJDSCHRIFTEN, RIJSWIJK., NL ISSN: 0032-4108
- C.HERKT-MAETZKY: "Kunststoffteile mit Laser beschriften" KUNSTSTOFFE., vol. 81, no. 4, April 1991 (1991-04), pages 341-346, XP000287477 CARL HANSER VERLAG. MUNCHEN., DE ISSN: 0023-5563

## Description

### Technical Field

The present invention relates to a method of making an odometer wheel and marking a character on the surface of an odometer wheel.

### Background to Invention

It is well known to treat patients with medicaments contained in an aerosol, for example, in bronchodilator therapy. It is also known to use for such therapy medicaments which are contained in an aerosol and are administered to a patient by means of an inhalation device. The aerosol containers used in such inhalation devices are designed to deliver a predetermined dose of medicament upon each actuation and are known as Metered Dose Inhalers (MDI); see Peter Byron, Respiratory Drug Delivery, CRC Press, Boca Raton, FL (1990) for a general background on this form of therapy. Dose indicating devices, which typically count the number of doses delivered from or remaining in the aerosol container, can be used in MDI to enable patients to determine how much medicament is available in the container for future use.

Odometer wheels may be used as components of a dose counter in a MDI, as described in PCT application WO 98/56444. The dimensions of such odometer wheels are very small, typically of the order of 3 mm to 20 mm in diameter and 1 mm to 3 mm in thickness. Marking the surface of such wheels can therefore present several technical difficulties in terms of the ability to mark small, curved surfaces with distinct symbols. The definition and contrast of the characters on the wheel surface is very important, as the patient must be able to read these characters clearly. The characters should be indelibly marked on the surface of the wheels as they must remain clearly visible throughout the lifetime of the counter. Furthermore, it is desirable that the characters should not be raised above the wheel surface as this would not only increase wheel size but might also lead to increased friction during operation, resulting in wear and erosion of the symbols.

The applicants have now found that these problems can be overcome by the use of laser technology and photosensitive pigments. The present invention teaches a method whereby an odometer wheel is marked with a character by means of applying laser energy onto a photosensitive pigment incorporated or coated onto the surface of the wheel. This method has many advantages over conventional printing or etching techniques in that it can provide a high-speed process for permanently marking symbols onto the curved surface of the durable materials constituting the wheel. Unlike conventional printing techniques, this method is extremely accurate in marking characters of millimetre dimensions onto small curved surfaces, requires no printing dyes or inks and results in a very low reject rate of the final marked product. Furthermore, by modifying the surface chemistry of the pigments, excellent contrast and definition of the characters can be achieved, thereby enhancing their visibility and distinctiveness to the patient. Additional advantages accruing from this method are that the marked characters are not raised above the surface of the wheel and thus can neither be worn away nor lead to increased friction during operation.

Although laser marking systems are known In the art and a variety of techniques are employed, nowhere is there any teaching relating to the application of this technology to the marking of odometer wheels.

In the most common application, laser light is used to mark a symbol or character on the surface of an object by writing each character in a script style. Thus WO 98/17433 teaches a method for treating a portion of a surface with a pulsed scanning laser beam, thereby marking it with a series of dots which have the appearance of a smooth line from a distance. A variant of this approach involves the stencilling of each symbol or character by a pulsed laser beam by means of a mask wheel assembly.

US 4,803,336 discloses a 'High Speed Laser Marking System' whereby the images of symbols are used to reflect laser light onto the target surface from an antireflective wheel apparatus. A similar laser technique is described in GB 2218219A which involves the use of a mask assembly incorporating reflective portions to define images for marking.

Further laser systems and techniques are disclosed in:-
- EP-A-0 190 997
- 'Industriall Lasergraveren baant zich een weg', Wijers, Polytechnisch Tijdschrift, Werktnigbanw, Vol. 46, No. 1, January 1991, (1991-01), pp. 40-44 (ISSN: 0032-4108)
- 'Kunststoffteile mit Laser beschriften', Herkt-Maetzky, Kunststoff, Vol. 81, No. 4, April 1991 (1991-04), pp. 341-346 (ISSN: 0023-5563)

### Summary of Invention

According to one aspect of the present invention there is provided a method for making an odometer wheel comprising mixing a pigment with a polymeric material, the pigment having a colour which is modifiable by application of laser energy, and forming the wheel from the resultant mixture. The pigment is a photosensitive pigment or a heat-sensitive pigment that changes coloration in response to temperature change, for example in response to elevation of temperature caused by application of laser energy.

Suitably, the pigment is incorporated throughout the wheel.

In one aspect, the method comprises extruding the pigment with the polymeric material and injection moulding the resultant mixture to form the wheel.

In another aspect, the method additionally comprises applying a laser beam to the surface of the wheel to define a character thereon.

According to another aspect of the present invention there is provided a method for marking a surface of an odometer wheel with a character comprising:
coating or painting at least a portion of the surface of the wheel with a pigment, the pigment having a colour which is modifiable by application of laser energy; and applying a laser beam to the pigment at the surface to define the character.

In one aspect, the wheel comprises a polymeric material; this material may be an organic polymeric material, preferably an acetal based polymeric material.

In another aspect, the pigment is modifiable from a black or dark coloration to a white or light coloration by application of the laser energy.

In a further aspect, the pigment is modifiable from a white or light coloration to a black or dark coloration by application of the laser energy.

Suitably, the laser source can be any source suitable for laser marking, including Q-switched Neodymium Yttrium Aluminium Gamate, carbon dioxide, diode, fibre and copper vapour laser sources.

In one aspect, the laser energy is applicable in continuous fashion.

In another aspect, the laser energy is applicable is pulsed fashion.

Suitably, the laser source has a pulse width of from 0.5 to 20 microseconds.

Preferably the laser energy generated in the method is of wavelength 800 to 1200 nm and energy output 40 to 80 W. More preferably the laser energy is generated by a Q-switched Neodymium Yttrium Aluminium Garnate laser source of wavelength 1064 nm, frequency 100 Hz to 30 kHz and average output 65 W.

In one aspect, the method comprises defining the character by moving the laser beam across the surface in a controllable manner.

In another aspect, the method comprises moving the laser beam across the surface by reflecting the beam from a movable reflective surface.

In a further aspect, the method comprises moving the laser beam across the surface by using a galvanometer scanner to deflect the beam onto the surface of a focussing lens and subsequently reflecting the beam onto the odometer wheel surface.

Alternatively there is provided a method comprising defining the character by projecting the laser beam through a movable template. Preferably the template has a non-reflective surface. More preferably the template has an aperture defining the character.

In one aspect, the method comprises defining the character by projecting the laser beam onto a movable, non-reflective template bearing a reflective character and subsequently reflecting the beam onto the odometer surface.

In a further aspect, the method comprises defining the character by projecting the laser beam onto the surface of the odometer wheel and subsequently moving the wheel in a controllable manner.

In another aspect, the method comprises defining the character by projecting the laser beam onto a movable mirror which reflects the beam onto a static mirror and thence onto the wheel surface in a controllable manner.

In another embodiment of the present invention there is provided a method wherein the diameter of the wheel is in the range of 3 mm to 20 mm. Preferably the diameter of the wheel is in the range of 4 mm to 12 mm, more preferably being in the range of 6 mm to 10 mm in diameter.

In a further embodiment of the present invention there is provided a method wherein the thickness of the wheel is in the range of 1 mm to 3 mm.

In another embodiment of the present invention there is provided an odometer wheel obtainable by the method herein described.

The invention further provides a method for marking an odometer wheel substantially as described and shown in the accompanying drawings.

In another aspect of the present invention there is provided a dose counter for use in a metered dose inhaler comprising an odometer wheel within a dose counting mechanism as herein described.

In a further aspect of the present invention there is provided an aerosol container for use in a metered dose inhaler additionally comprising a dose counter as herein described attached thereto.

In yet another aspect of the present invention there is provided a metered dose inhaler comprising an actuator housing and receivable therein an aerosol container as herein described.

In another aspect of the present invention there is provided a kit of parts comprising an actuator housing and an aerosol container as herein described.

### Brief Description of Drawings

The invention will now be described with reference to the accompanying drawings in which:
Figure 1 is a perspective view of an odometer wheel according to the invention.
Figure 2 is a schematic representation of an odometer wheel being marked by direct irradiation with a laser beam according to the invention.
Figure 3 is a schematic representation of an odometer wheel being marked by irradiation with a laser beam following deflection of the beam from a movable reflective surface according to the invention.
Figure 4 is a schematic representation of an odometer wheel being marked by irradiation with a laser beam using a galvanometer scanner to deflect the beam into a focussing lens and thence onto the odometer wheel surface according to the invention.
Figure 5 is a schematic representation of an odometer wheel being marked by irradiation with a laser beam, the character being defined by irradiating the beam through an aperture in a movable template according to the invention.
Figure 6 is a schematic representation of an odometer wheel being marked by irradiation with a laser beam, the character being defined by reflecting the beam from the reflective surface of a character on a non-reflective template according to the invention.
Figure 7 is a schematic representation of an odometer wheel being marked by irradiation with a laser beam, the character being defined by reflecting the beam from a movable mirror onto the surface of a static mirror and onwards onto the surface of the wheel according to the invention.

### Detailed Description of Drawings

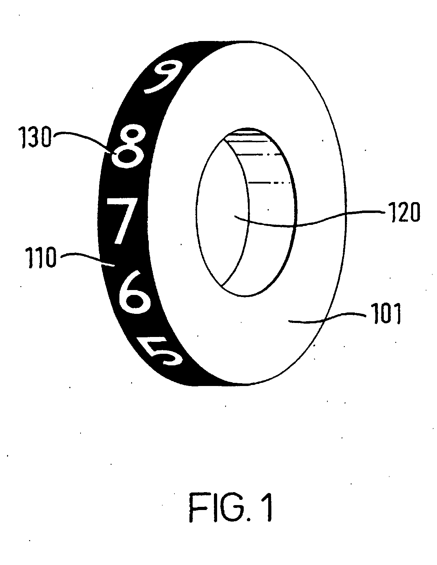

An odometer wheel 101 made by the method according to the invention is shown in Figure 1. The wheel 101 comprises a surface 110 which is clearly visible to the patient in use and characters 130 denoting the number of doses delivered or remaining in the aerosol container of the MDI. A dose counter (not shown) may attach to the wheel 101 through its centre 120; alternatively an axle (not shown), connected to the wheel 101 through its centre 120, may be used to rotate or move the wheel 101 during the marking process described below. The odometer wheel 101 has a photosensitive pigment incorporated within it or the surface may be coated or painted with the photosensitive pigment.

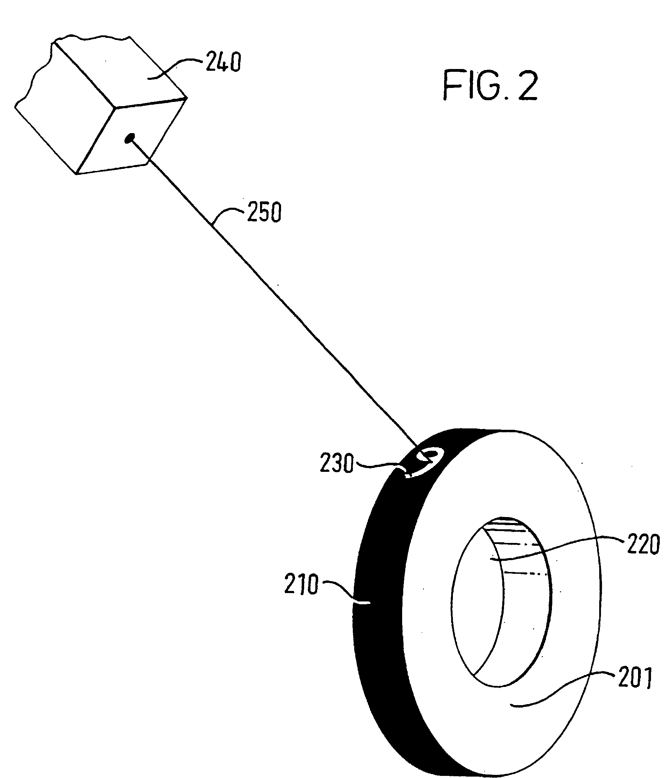

Figure 2 depicts the marking of character 230 on the surface 210 of the wheel 201. The wheel 201 contains a photosensitive pigment incorporated within it or coated or painted over its surface 210. The wheel 201 is rotated through a predetermined angle by means of an axle (not shown), attached through its centre 220, to expose the surface for marking by the laser. Following irradiation with a laser 240 generated light beam 250 the pigment is modified from a black or dark coloration to a white or light coloration. By moving the laser beam across the surface 210 of the wheel 201 the character 230 is defined. In an alternative method for defining the character 230, the wheel 201 is moved in a controllable manner during irradiation by the beam 250 by means of a rod (not shown) attached through its centre 220.

The laser beam is of wavelength 800 nm to 1200 nm and of energy output 40 W to 80 W. Preferably the laser beam is generated by a Q-switched Neodymium Yttrium Aluminium Garnate laser source of wavelength 1064 nm, frequency 100 Hz to 30 kHz and average output 65 W. The operation of such lasers is well known in the art.

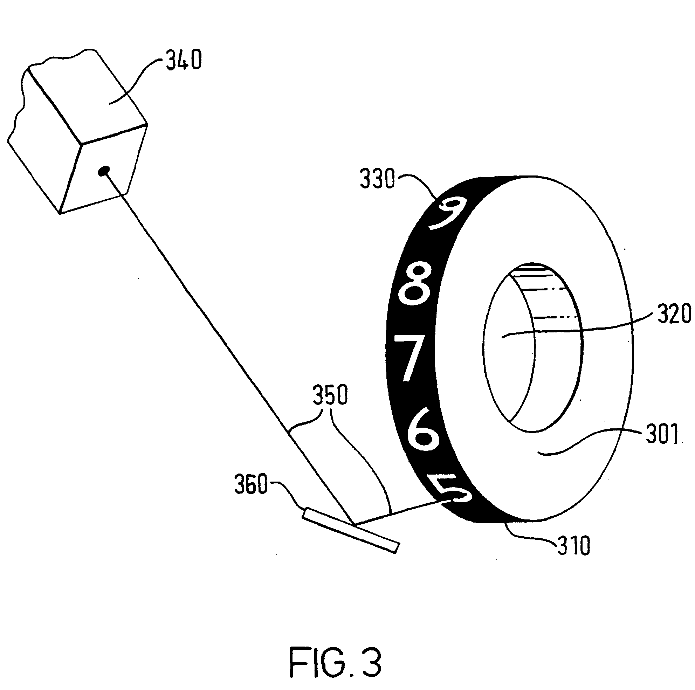

The character 330 may be defined as illustrated in Figure 3. The wheel 301 contains a photosensitive pigment incorporated within it or coated or painted over its surface 310. Laser source 340 emits a beam 350 which is reflected from a movable reflective surface 360 onto the surface 310 of the wheel 301. The controlled movement of the reflective surface 360 directs the reflection of the beam 350 across the surface of the wheel 310 and thereby defines the character 330. The wheel 301 is rotated through a predetermined angle by means of an axle (not shown), attached through its centre 320, to expose the surface 310 for marking by the laser beam 350.

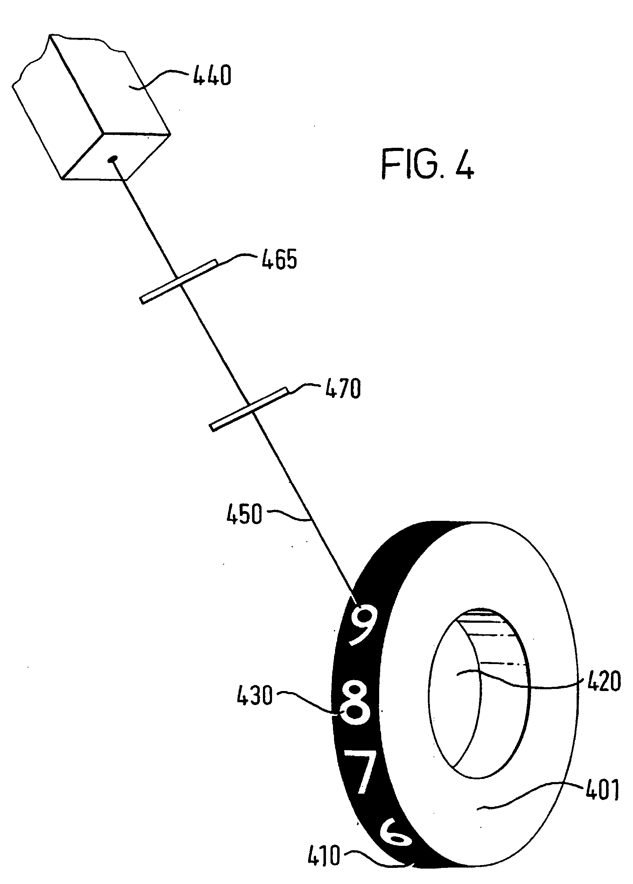

Alternatively the character 430 may be defined as shown in Figure 4. The wheel 401 contains a photosensitive pigment incorporated within it or coated or painted over its surface 410. Laser beam 450, generated by source 440, is deflected into a focussing lens 470 by a galvanometer scanner 465 and then focussed onto the surface 410 of the wheel 401 thereby defining the character 430. The wheel 401 is rotated through a predetermined angle by means of an axle (not shown), attached through its centre 420, to expose the surface 410 for marking by the laser beam 450.

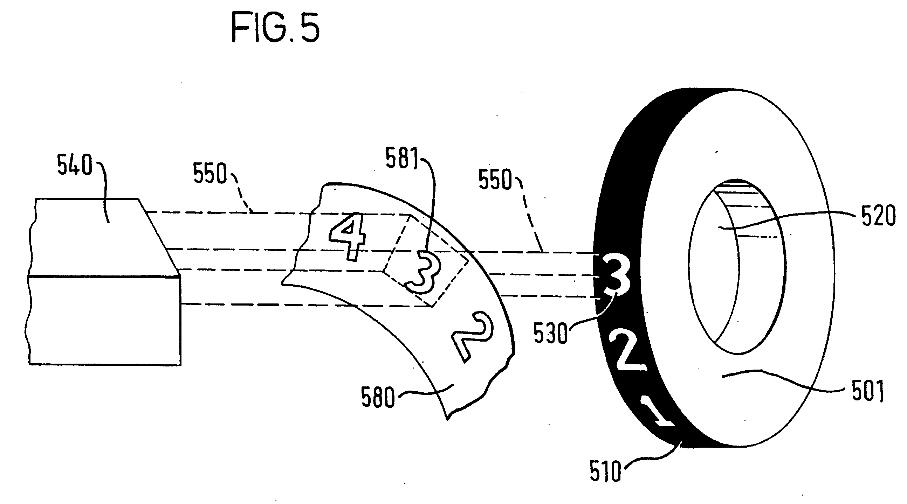

Figure 5 depicts the use of the template 580 in defining the character 530. The wheel 501 contains a photosensitive pigment incorporated within it or coated or painted over its surface 510. The laser beam 550, generated by laser source 540, is projected onto a movable template 580 comprising an aperture 581 delimiting the character 530. The template may be made of any material which is opaque to the laser beam 550 and is suitable for the formation of a thin sheet, such as copper, aluminium or tungsten. The beam 550 is projected through the aperture 581 onto the surface 510 of the wheel 501 thereby defining the character 530. The character, defined by aperture 581, is selected by movement of the template 580. Wheel 501 is rotated through a predetermined angle by an axle (not shown), attached through its centre 520, to expose the surface 510 for marking with the character 530 by the laser beam 550.

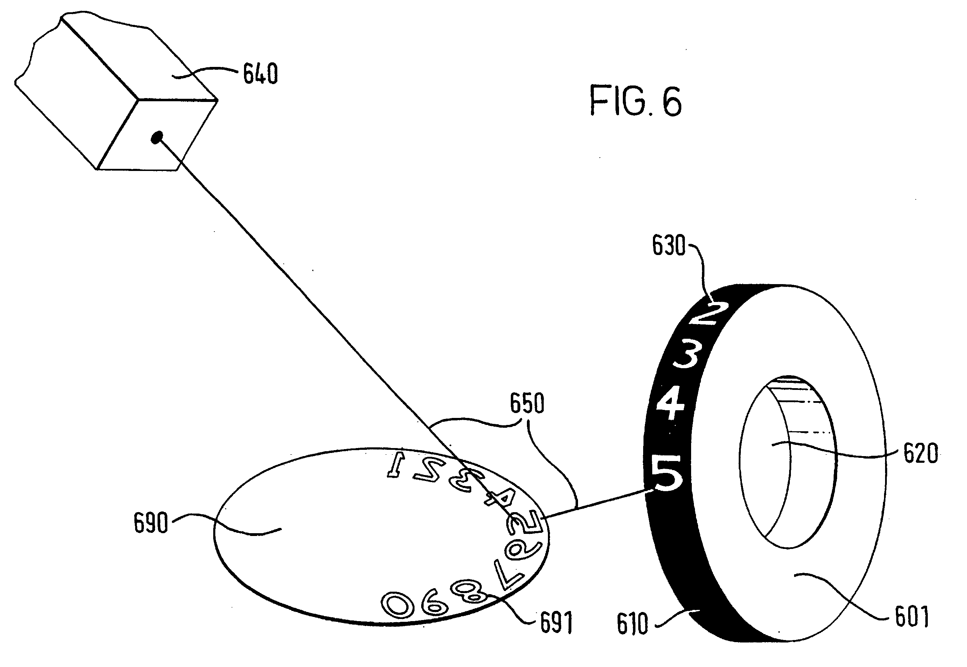

The character 630 may be defined as illustrated in Figure 6. The wheel 601 contains a photosensitive pigment incorporated within it or coated or painted over its surface 610. The laser beam 650, generated by source 640, is projected onto a movable, non-reflective template 690 bearing the reflective character 691 and is then reflected onto the surface 610 of the wheel 601 thereby defining the character 630. The template 690 may be metallic in nature with an anti-reflective coating whilst the reflective character 691 may be a mirrored surface level with, or raised above, the surface of the template 690. The wheel 601 is rotated through a predetermined angle by means of a axle (not shown), attached through its centre 620, to expose the surface 610 for marking by the laser beam 650.

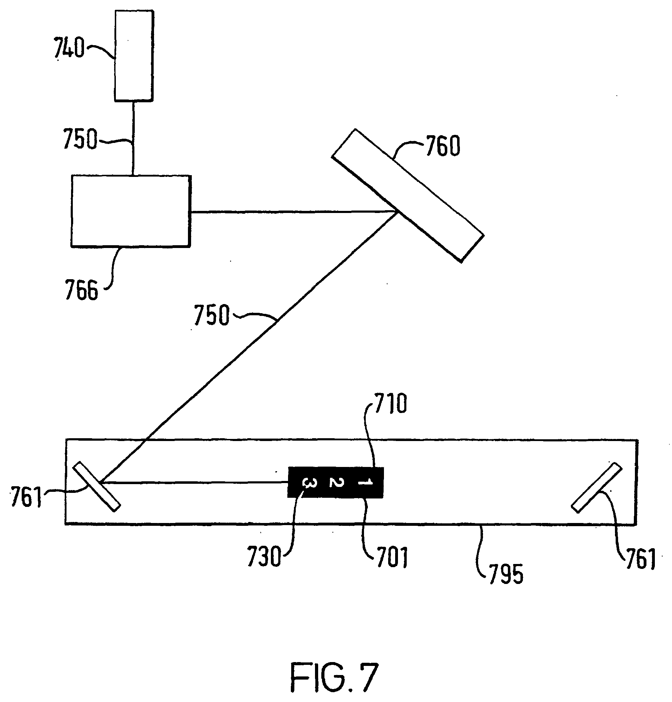

Figure 7 illustrates the marking of character 730 on wheel 701. The wheel 701 contains a photosensitive pigment incorporated within it or coated or painted over its surface 710. The source 740 projects pulsed beam 750 onto a movable mirror 760 by means of a galvanic lens 766; the beam is further deflected onto a fixed mirror 761, positioned in a carrousel 795, and then onto the surface 710 of the wheel 701. The movement of mirror 760 in a controllable manner defines the character 730 on the surface 710 of the wheel 701. The wheel 701 remains static within the carrousel 795 which contains a plurality of static mirrors 761. Irradiation of different static mirrors 761 within the carousel 795 by beam 750 results in different characters 730 being marked on the surface 710.

Photosensitive pigments, which can be used according to the present invention, are known in the art. Suitable pigments are commercially available, such as the range of Iriodin® LS laser markable pigments from Merck Company. Other pigments are disclosed in the patent literature.

US patent 4,861,620 describes a number of pigments that change colour at elevated temperatures and could be utilised in the method described above. Cobalt based pigments such as cobalt oxalates, formates and phosphates, together with mixtures of cobalt and nickel oxalates are particularly suitable, as is potassium cobalticnitride. Similarly a number of pigments based upon mercury, copper, bismuth, nickel and lead could have utility in the present invention. Of particular relevance to the present invention is the use of pigments of bismouth oxalate which change coloration from white to grey/black on heating at temperatures in excess of 180 C.

Pigments which discolour from black to white on laser irradiation are disclosed in US patent 5,853,955. A combination pigment composed of oxides of barium, calcium, cobalt and boron is prepared by adding powdered calcium sulphate pigment to a sodium borate solution then neutralising with cobalt and barium chloride to precipitate cobalt and barium borate onto the pigment. The dried combination pigment, which is black in colour on incorporation into an epoxy resin, discolours to white on laser irradiation.

Odometer wheels herein described are suitable for use in dose counters, particularly those used in medicament dispensers. Dose counters of the present invention are suitable for use in medicament dispensers which administer predefined doses of dry powder, liquid or aerosol medicaments to patients. The dose counters find particular utility in aerosol medicament dispensers or inhalation devices which deliver specified quantities of drugs for treatment of bronchial disorders. Such inhalation devices comprise an aerosol canister containing a medicament and a tubular housing or sleeve in which the aerosol canister or container is located and an outlet tube leading out from the tubular housing. The aerosol containers used in such inhalation devices are designed to deliver a predetermined dose of medicament upon each actuation by means of an outlet valve member at one end which can be opened either by depressing the valve member while the container is held stationary or by depressing the container while the valve member is held stationary. In the use of such devices, which are known as metered dose inhalers, the aerosol container is placed in the tubular housing with the outlet valve member of the container communicating via a support with the outlet tube, for example a nozzle or mouthpiece. When used for dispensing medicaments, for example in bronchodilation therapy, the housing is then held by the patient in a more or less upright condition and the mouthpiece or nozzle of the inhalation device is placed in the mouth or nose of the patient.

The aerosol container is pressed towards the support to dispense a dose of medicament from the container which is then inhaled by the patient.

The dose counter of the present invention may be attached to the aerosol container or to the housing such that the odometer wheels are easily visible to the patient. On actuation of the device, by pressing the container towards the support, the dose counter mechanism rotates the odometer wheel through a predefined number of degrees. In this manner, the number of doses used or remaining within the aerosol container is displayed to the patient on the odometer wheel. The use of such a dose counter with a metered dose inhalation device is disclosed in PCT application WO 98/56444.

The dose counter of the present invention is suitable for use in a dispenser for dispensing medicament, particularly for the treatment of respiratory disorders. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate, ketotifen or nedocromil; antiinfectives e.g., cephalosporins, penicillins, streptomycin, sulphonamides, tetracyclines and pentamidine; antihistamines, e.g., methapyrilene; anti- inflammatories, e.g., beclomethasone dipropionate, fluticasone propionate, flunisolide, budesonide, rofleponide, mometasone furoate or triamcinolone acetonide; antitussives, e.g., noscapine; bronchodilators, e.g., albuterol, salmeterol, ephedrine, adrenaline, fenoterol, formoterol, isoprenaline, metaproterenol, phenylephrine, phenylpropanolamine, pirbuterol, reproterol, rimiterol, terbutaline, isoetharine, tulobuterol, or (-)-4-amino-3,5-dichloro-α-[[[6-[2-(2-pyridinyl)ethoxy] hexyl]methyl] benzenemethanol; diuretics, e.g., amiloride; anticholinergics, e.g., ipratropium, tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclometasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) in combination with an antiinflammatory steroid such as a beclomethasone ester (e.g., the dipropionate) or a fluticasone ester (e.g., the propionate). A particularly preferred combination comprises salmeterol xinafoate salt and fluticasone propionate.

The medicaments may be delivered in the form of a pharmaceutical aerosol formulation comprising a medicament and a fluorocarbon or hydrogen-containing chlorofluorocarbon propellant.

Suitable propellants include, for example, C₁₋₄hydrogen-containing chlorofluorocarbons such as CH₂ClF, CClF₂CHClF, CF₃CHClF, CHF₂CClF₂, CHClFCHF₂, CF₃CH₂Cl and CClF₂CH₃; C₁₋₄hydrogen-containing fluorocarbons such as CHF₂CHF₂, CF₃CH₂F, CHF₂CH₃ and CF₃CHFCF₃; and perfluorocarbons such as CF₃CF₃ and CF3CF₂CF₃.

Where mixtures of the fluorocarbons or hydrogen-containing chlorofluorocarbons are employed they may be mixtures of the above identified compounds or mixtures, preferably binary mixtures, with other fluorocarbons or hydrogen-containing chloro- fluorocarbons for example CHClF₂, CH₂F₂ and CF₃CH₃. Preferably a single fluorocarbon or hydrogen-containing chlorofluorocarbon is employed as the propellant. Particularly preferred as propellants are C₁₋₄hydrogen-containing fluorocarbons such as 1,1,1,2- tetrafluoroethane (CF₃CH₂F) and 1,1,1,2,3,3,3-heptafluoro-n-propane (CF₃CHFCF₃) or mixtures thereof.

The pharmaceutical formulations for use in the canisters of the invention contain no components which provoke the degradation of stratospheric ozone. In particular the formulations are substantially free of chlorofluorocarbons such as CCl₃F, CCl₂F₂ and CF₃CCl₃.

The propellant may additionally contain a volatile adjuvant such as a saturated hydrocarbon for example propane, n-butane, isobutane, pentane and isopentane or a dialkyl ether for example dimethyl ether. In general, up to 50% w/w of the propellant may comprise a volatile hydrocarbon, for example 1 to 30% w/w. However, formulations which are free or substantially free of volatile adjuvants are preferred. In certain cases, it may be desirable to include appropriate amounts of water, which can be advantageous in modifying the dielectric properties of the propellant.

The invention is particularly useful with propellants (including propellant mixtures) which are more hygroscopic than P11, P114 and/or P12 such as HFA-134a and HFA-227.

A polar co-solvent such as C₂₋₆ aliphatic alcohols and polyols e.g. ethanol, isopropanol and propylene glycol, preferably ethanol, may be included in the drug formulation in the desired amount to improve the dispersion of the formulation, either as the only excipient or in addition to other excipients such as surfactants. Suitably, the drug formulation may contain 0.01 to 5% w/w based on the propellant of a polar co-solvent e.g. ethanol, preferably 0.1 to 5% w/w e.g. about 0.1 to 1% w/w.

A surfactant may also be employed in the aerosol formulation. Examples of conventional surfactants are disclosed in EP-A-372,777. The amount of surfactant employed is desirable in the range 0.0001% to 50% weight to weight ratio relative to the medicament, in particular, 0.05 to 5% weight to weight ratio. Preferred surfactants are lecithin, oleic acid and sorbitan trioleate. Preferred formulations, however, are free or substantially free of surfactant.

Pharmaceutical formulations may contain 0.0001 to 50% w/w, preferably 0.001 to 20%, for example 0.001 to 1% of sugar relative to the total weight of the formulation. Generally the ratio of medicament to sugar falls within the range of 1:0.01 to 1:100 preferably 1:0.1 to 1:10. Typical sugars which may be used in the formulations include, for example, sucrose, lactose and dextrose, preferably lactose, and reducing sugars such as mannitol and sorbitol, and may be in micronised or milled form.

The final aerosol formulation desirably contains 0.005-10% w/w, preferably 0.005 to 5% w/w, especially 0.01 to 1.0% w/w, of medicament relative to the total weight of the formulation.

It may be appreciated that any of the parts of the dispenser which contact the medicament may be coated with materials such as fluoropolymer materials which reduce the tendency of medicament to adhere thereto. Suitable fluoropolymers include polytetrafluoroethylene (PTFE) and fluoroethylene propylene (FEP). Any movable parts may also have coatings applied thereto which enhance their desired movement characteristics. Frictional coatings may therefore be applied to enhance frictional contact and lubricants used to reduce frictional contact as necessary.

It will be understood that the present disclosure is for the purpose of illustration only and the invention extends to modifications, variations and improvements thereto within the scope of the appended claims.

## Claims

1. A method for making an odometer wheel (101; 201; ... 701) comprising mixing a pigment with a polymeric material, said pigment having a colour which is modifiable by application of laser energy, and forming said wheel from the resultant mixture.

2. A method according to claim 1 comprising extruding said pigment with said polymeric material and injection moulding the resultant mixture to form the wheel.

3. A method according to claim 2 comprising the additional step of applying a laser beam (250; ... 750) to the surface (110; 210; ... 710) of the wheel to define a character (130; 230; ... 730) thereon.

4. A method for marking a surface (110; ... 710) of an odometer wheel (101; ...701) with a character (130; ... 730) comprising:
coating or painting at least a portion of the surface of said wheel with a pigment, said pigment having a colour which is modifiable by application of laser energy; and
applying a laser beam (250; ...750) to the pigment at the surface to define said character.

5. A method according to claim 4 wherein said wheel comprises a polymeric material.

6. A method according to any of claims 1 to 3 or 5 wherein said polymeric material is an organic polymeric material, preferably an acetal based polymeric material.

7. A method according to any of claims 1 to 6 wherein the pigment is modifiable from a black or dark coloration to a white or light coloration by application of the laser energy.

8. A method according to any of claims 1 to 6, wherein the pigment is modifiable from a white or light coloration to a black or dark coloration by the application of the laser energy.

9. A method according to any of claims 1 to 8, wherein a laser source (240; ... 740) generates the laser energy, said source being any source suitable for laser marking, including Q-switched Neodymium Yttrium Aluminium Gamate, carbon dioxide, diode, fibre and copper vapour laser sources.

10. A method according to any of claims 3 to 5, or to any of claims 6 to 9 when appended to any of claims 3 to 5, wherein the laser energy is applied in continuous fashion.

11. A method according to any of claims 3 to 5, or to any of claims 6 to 9 when appended to any of claims 3 to 5, wherein the laser energy is applied in pulsed fashion.

12. A method according to claim 11, wherein the laser source has a pulse width of from 0.5 to 20 µs.

13. A method according to any of claims 3 to 5, or to any of claims 6 to 9 when appended to any of claims 3 to 5, or to any of claims 10 to 12 comprising generating the laser energy at a wavelength of 800 to 1200 nm and with an energy output of 40 to 80 W.

14. A method according to claim 13 wherein the laser energy is generated by a Q-switched Neodymium Yttrium Aluminium Gamate laser source of wavelength 1064 nm, frequency 100 Hz to 30 kHZ and average output 65 W.

15. A method according to any of claims 3 to 5, or to any of claims 6 to 9 when appended to any of claims 3 to 5, or to any of claims 10 to 14 comprising defining the character by moving said laser beam across the surface in a controllable manner.

16. A method according to claim 15 comprising moving the laser beam across the surface by reflecting the beam from a movable reflective surface (360; 690; 760).

17. A method according to claim 15 comprising moving the laser beam across the surface by using a galvanometer scanner (465) to deflect the beam onto the surface of a focussing lens (470) and subsequently reflecting the beam onto the odometer wheel surface.

18. A method according to any of claims 3 to 5, or to any of claims 6 to 9 when appended to any of claims 3 to 5, or to any of claims 10 to 14 comprising defining the character by projecting the laser beam through a movable template (580; 690).

19. A method according to claim 18 wherein said template (580) has an aperture (581) defining the character.

20. A method according to any of claims 3 to 5, or to any of claims 6 to 9 when appended to any of claims 3 to 5, or to any of claims 10 to 14 comprising defining the character by projecting the laser beam onto a movable, non-reflective template (690) bearing a reflective character (691) and subsequently reflecting the beam onto the odometer surface.

21. A method according to any of claims 3 to 5, or to any of claims 6 to 9 when appended to any of claims 3 to 5, or to any of claims 10 to 14 comprising defining the character by projecting the laser beam onto the surface of the odometer wheel and subsequently moving said wheel in a controllable manner.

22. A method according to any of claims 3 to 5, or to any of claims 6 to 9 when appended to any of claims 3 to 5, or to any of claims 10 to 14 comprising defining the character by projecting the laser beam onto a movable mirror (760) which reflects the beam onto a static mirror (761) and thence onto the wheel surface in a controllable manner.

23. A method according to any of claims 1 to 22 wherein the diameter of the wheel is in the range of 3 mm to 20 mm.

24. A method according to claim 23 wherein the diameter of the wheel is in the range of 4 mm to 12 mm.

25. A method according to either of claims 23 or 24 wherein the diameter of the wheel is in the range of 6 mm to 10 mm.

26. A method according to any of claims 1 to 25 wherein the thickness of the wheel is in the range of 1 mm to 3 mm.

27. Odometer wheel (101; ... 701) obtainable by the method of any of claims 1 to 26.

28. Dose counter for use in a metered dose inhaler comprising an odometer wheel (101; ... 701) according to claim 27 within a dose counting mechanism.

29. Aerosol container for use in a metered dose inhaler additionally comprising a dose counter according to claim 28 attached thereto.

30. Metered dose inhaler comprising an actuator housing and receivable therein an aerosol container according to claim 29.

31. Kit of parts comprising an actuator housing and an aerosol container according to claim 29.

## Patentansprüche

1. Verfahren zum Herstellen eines Messrades (101; 201; .. 701), umfassend Mischen eines Pigments mit einem Polymerwerkstoff, wobei das Pigment eine Farbe aufweist, die durch Aufbringen von Laserenergie modifizierbar ist, und Bilden des Rades aus der resultierenden Mischung.

2. Verfahren ach Anspruch 1, umfassend Extrudieren des Pigments mit dem Polymerwerkstoff und Spritzgießen der resultierenden Mischung, um das Rad zu bilden.

3. Verfahren nach Anspruch 2, umfassend den zusätzlichen Schritt des Aufbringens eines Laserstrahls (250; .. 750) auf die Oberfläche (110; 210; .. 710) des Rades um ein Schriftzeichen (130; 230; .. 730) darauf zu definieren.

4. Verfahren zum Markieren einer Oberfläche (110; .. 710) eines Messrades (101; .. 701) mit einem Schriftzeichen (130; .. 730) umfassend:
Beschichten oder Bestreichen zumindest eines Abschnitts der Oberfläche des Rades mit einem Pigment, wobei das Pigment eine Farbe aufweist, die durch Aufbringen von Laserenergie modifizierbar ist; und
Aufbringen eines Laserstrahls (250; .. 750) auf das Pigment an der Oberfläche, um das Schriftzeichen zu definieren.

5. Verfahren nach Anspruch 4, wobei das Rad einen Polymerwerkstoff umfasst.

6. Verfahren nach einem der Ansprüche 1 bis 3 oder 5, wobei der Polymerwerkstoff ein organischer Polymerwerkstoff ist, vorzugsweise ein auf Acetal basierender Polymerwerkstoff.

7. Verfahren nach einem der Ansprüche 1 bis 6, wobei das Pigment durch Aufbringen der Laserenergie von einer schwarzen oder dunklen Färbung zu einer weißen oder hellen Färbung modifizierbar ist.

8. Verfahren nach einem der Ansprüche 1 bis 6, wobei das Pigment durch Aufbringen der Laserenergie von einer weißen oder hellen Färbung zu einer schwarzen oder dunklen Färbung modifizierbar ist.

9. Verfahren nach einem der Ansprüche 1 bis 8, wobei eine Laserquelle (240; .. 740) die Laserenergie erzeugt, wobei die Quelle eine beliebige Quelle ist, die zum Lasermarkieren geeignet ist, umfassend Q-switched Neodymium Yttrium Aluminium Garnat-, Kohlendioxid-, Dioden-, Faser- und Kupferdampflaserquellen.

10. Verfahren nach einem der Ansprüche 3 bis 5, oder einem der Ansprüche 6 bis 9, wenn abhängig von einem der Ansprüche 3 bis 5, wobei die Laserenergie in einer kontinuierlichen Art aufgebracht wird.

11. Verfahren nach einem der Ansprüche 3 bis 5 oder einem der Ansprüche 6 bis 9, wenn abhängig von einem der Ansprüche 3 bis 5, wobei die Laserenergie in einer gepulsten Art aufgebracht wird.

12. Verfahren nach Anspruch 11, wobei die Laserquelle eine Impulsbreite von 0,5 bis 20 Mikrosekunden aufweist.

13. Verfahren nach einem der Ansprüche 3 bis 5 oder einem der Ansprüche 6 bis 9, wenn abhängig von einem der Ansprüche 3 bis 5 oder nach einem der Ansprüche 10 bis 12, umfassend Erzeugen der Laserenergie mit einer Wellenlänge von 800 bis 1200 nm und mit einer Energieausgangsleistung von 40 bis 80 W.

14. Verfahren nach Anspruch 13, wobei die Laserenergie durch eine Q-switched Neodymium Yttrium Aluminium Garnatlaserquelle mit einer Wellenlänge von 1064 nm, einer Frequenz von 100 Hz bis 30 kHz und einer durchschnittlichen Ausgangsleistung von 65 W erzeugt wird.

15. Verfahren nach einem der Ansprüche 3 bis 5 oder nach einem der Ansprüche 6 bis 9, wenn abhängig von einem der Ansprüche 3 bis 5 oder nach einem der Ansprüche 10 bis 14, umfassend Definieren des Schriftzeichens durch Bewegen des Laserstrahls über die Oberfläche in einer kontrollierten Art.

16. Verfahren nach Anspruch 15, umfassend Bewegen des Laserstrahls über die Oberfläche durch Reflektieren des Strahls von einer beweglichen reflektierenden Oberfläche (360; 690; 760).

17. Verfahren nach Anspruch 15, umfassend Bewegen des Laserstrahls über die Oberfläche unter Verwendung eines Galvanometer-Abtasters (465), um den Strahl auf die Oberfläche einer Fokussierlinse (470) abzulenken und den Strahl nachfolgend auf die Oberfläche des Messrades zu reflektieren.

18. Verfahren nach einem der Ansprüche 3 bis 5 oder nach einem der Ansprüche 6 bis 9, wenn abhängig von einem der Ansprüche 3 bis 5, oder nach einem der Ansprüche 10 bis 14, umfassend Definieren des Schriftzeichens durch Projizieren des Laserstrahls durch eine bewegliche Schablone (580; 690).

19. Verfahren nach Anspruch 18, wobei die Schablone (580) eine Öffnung (581) aufweist, die das Schriftzeichen definiert.

20. Verfahren nach einem der Ansprüche 3 bis 5, oder nach einem der Ansprüche 6 bis 9, wenn abhängig von einem der Ansprüche 3 bis 5 oder nach einem der Ansprüche 10 bis 14, umfassend Definieren des Schriftzeichens durch Projizieren des Laserstrahls auf eine bewegliche, nicht reflektierende Schablone (690), die ein reflektierendes Schriftzeichen (691) trägt und nachfolgend Reflektieren des Strahls auf die Oberfläche des Messrades.

21. Verfahren nach einem der Ansprüche 3 bis 5 oder nach einem der Ansprüche 6 bis 9, wenn abhängig von einem der Ansprüche 3 bis 5 oder nach einem der Ansprüche 10 bis 14, umfassend Definieren des Schriftzeichens durch Projizieren des Laserstrahls auf die Oberfläche des Messrades und nachfolgend Bewegen des Rades in einer kontrollierten Art.

22. Verfahren nach einem der Ansprüche 3 bis 5 oder nach einem der Ansprüche 6 bis 9, wenn abhängig von einem der Ansprüche 3 bis 5 oder nach einem der Ansprüche 10 bis 14, umfassend Definieren des Schriftzeichens durch Projizieren des Laserstrahls auf einen beweglichen Spiegel (760), der den Strahl auf einen statischen Spiegel (761) reflektiert und darauffolgend auf die Radoberfläche in einer kontrollierten Art.

23. Verfahren nach einem der Ansprüche 1 bis 22, wobei der Durchmesser des Rades in einem Bereich von 3 mm bis 20 mm liegt.

24. Verfahren nach Anspruch 23, wobei der Durchmesser des Rades in einem Bereich von 4 mm bis 12 mm liegt.

25. Verfahren nach Anspruch 23 oder 24, wobei der Durchmesser des Rades in einem Bereich von 6 mm bis 10 mm liegt.

26. Verfahren nach einem der Ansprüche 1 bis 25, wobei die Dicke des Rades in einem Bereich von 1 mm bis 3 mm liegt.

27. Messrad (101; .. 701), welches durch das Verfahren nach einem der Ansprüche 1 bis 26 zu erlangen ist.

28. Dosiszähler für die Verwendung in einem Dosisinhalator, umfassend ein Messrad (101; .. 701) gemäß Anspruch 27 innerhalb eines Dosiszählmechanismus.

29. Aerosolbehälter für die Verwendung in einem Dosisinhalator, zusätzlich umfassend einen Dosiszähler gemäß Anspruch 28, der daran angebracht ist.

30. Dosisinhalator, umfassend ein Betätigungsgehäuse und darin aufnehmbar einen Aerosolbehälter gemäß Anspruch 29.

31. Teilezusammenstellung, umfassend ein Betätigungsgehäuse und einen Aerosolbehälter gemäß Anspruch 29.

## Revendications

1. Procédé de fabrication d'une roue de comptage (101 ; 201 ; ...701), comprenant le mélange d'un pigment avec une matière polymère, ledit pigment ayant une couleur qui est modifiable par application d'énergie laser, et la formation de ladite roue à partir du mélange résultant.

2. Procédé selon la revendication 1, comprenant l'extrusion dudit pigment avec ladite matière polymère et le moulage par injection du mélange résultant pour former la roue.

3. Procédé selon la revendication 2, comprenant l'étape additionnelle d'application d'un faisceau laser (250 ; ... 750) à la surface (110 ; 210 ; ...710) de la roue, pour définir un caractère (130 ; 230 ; ... 730) sur cette surface.

4. Procédé de marquage d'une surface (110 ; ... 710) d'une roue de comptage (101 ; ... 701) avec un caractère (130 ; ... 730), comprenant :
le revêtement ou la peinture d'au moins une portion de la surface de ladite roue avec un pigment, ledit pigment ayant une couleur qui est modifiable par application d'énergie laser ; et
l'application d'un faisceau laser (250 ; ... 750) au pigment, à la surface, pour définir le dit caractère.

5. Procédé selon la revendication 4, dans lequel la dite roue comprend une matière polymère.

6. Procédé selon une quelconque des revendications 1 à 3 ou 5, dans lequel la dite matière polymère est un polymère organique, de préférence un polymère à base d'acétal.

7. Procédé selon une quelconque des revendications 1 à 6, dans lequel le pigment est modifiable d'une coloration noire ou sombre à une coloration blanche ou claire par application de l'énergie laser.

8. Procédé selon une quelconque des revendications 1 à 6, dans lequel le pigment est modifiable d'une coloration blanche ou claire à une coloration noire ou sombre par l'application de l'énergie laser.

9. Procédé selon une quelconque des revendications 1 à 8, dans lequel une source laser (240 ;...740) engendre l'énergie laser, la dite source étant toute source convenant pour le marquage au laser, incluant des sources laser à déclenchement de type Néodyme Yttrium Aluminium Grenat, gaz carbonique, diode, fibre et vapeur de cuivre,

10. Procédé selon une quelconque des revendications 3 à 5 ou une quelconque des revendications 6 à 9 lorsqu'elle dépend d'une quelconque des revendications 3 à 5, dans lequel l'énergie laser est appliquée de façon continue.

11. Procédé selon une quelconque des revendications 3 à 5 ou une quelconque des revendications 6 à 9 lorsqu'elle dépend d'une quelconque des revendications 3 à 5, dans lequel l'énergie laser est appliquée de façon pulsée.

12. Procédé selon la revendication 11, dans lequel la source laser a une largeur d'impulsion de 0,5 à 20 µs.

13. Procédé selon une quelconque des revendications 3 à 5 ou une quelconque des revendications 6 à 9 lorsqu'elle dépend d'une quelconque des revendications 3 à 5, ou une quelconque des revendications 10 à 12, comprenant la génération de l'énergie laser à une longueur d'onde de 800 à 1200 nm et avec une sortie d'énergie de 40 à 80 W.

14. Procédé selon la revendication 13, dans lequel l'énergie laser est engendrée par une source laser à déclenchement de type Néodyme Yttrium Aluminium Grenat ayant une longueur d'onde de 1064 nm, une fréquence de 100 Hz à 30 kHz et une puissance moyenne de 65 W.

15. Procédé selon une quelconque des revendications 3 à 5 ou une quelconque des revendications 6 à 9 lorsqu'elle dépend d'une quelconque des revendications 3 à 5, ou une quelconque des revendications 10 à 14, comprenant la définition du caractère par déplacement dudit faisceau laser sur la surface d'une manière réglée.

16. Procédé selon la revendication 15, comprenant Je déplacement du faisceau laser sur la surface, par réflexion du faisceau au moyen d'une surface réfléchissante mobile (360 ; 690 ; 760).

17. Procédé selon la revendication 15, comprenant le déplacement du faisceau laser sur la surface au moyen d'un dispositif de balayage à galvanomètre (465) pour dévier le faisceau sur la surface d'une lentille de focalisation (470), puis la réflexion du faisceau vers la surface de la roue de comptage.

18. Procédé selon une quelconque des revendications 3 à 5 ou une quelconque des revendications 6 à 9 lorsqu'elle dépend d'une quelconque des revendications 3 à 5, ou une quelconque des revendications 10 à 14, comprenant la définition du caractère par projection du faisceau laser à travers un masque mobile (580 ; 690).

19. Procédé selon la revendication 18, dans lequel ledit masque (580) comporte une ouverture (581) définissant le caractère.

20. Procédé selon une quelconque des revendications 3 à 5 ou une quelconque des revendications 6 à 9 lorsqu'elle dépend d'une quelconque des revendications 3 à 5, ou une quelconque des revendications 10 à 14, comprenant la définition du caractère par projection du faisceau laser sur un gabarit non réfléchissant mobile (690) qui porte un caractère réfléchissant (691), puis réflexion du faisceau vers la surface de la roue de comptage.

21. Procédé selon une quelconque des revendications 3 à 5 ou une quelconque des revendications 6 à 9 lorsqu'elle dépend d'une quelconque des revendications 3 à 5, ou une quelconque des revendications 10 à 14, comprenant la définition du caractère par projection du faisceau laser sur la surface de la roue de comptage, puis déplacement de la dite roue d'une manière commandée.

22. Procédé selon une quelconque des revendications 3 à 5 ou une quelconque des revendications 6 à 9 lorsqu'elle dépend d'une quelconque des revendications 3 à 5, ou une quelconque des revendications 10 à 14, comprenant la définition du caractère par projection du faisceau laser sur un miroir mobile (760) qui réfléchit le faisceau vers un miroir statique (761) et, de là, vers la surface de la roue d'une manière commandée.

23. Procédé selon une quelconque des revendications 20 à 22, dans lequel le diamètre de la roue est dans la plage de 3 mm à 20 mm.

24. Procédé selon la revendication 23, dans lequel le diamètre de la roue est dans la plage de 4 mm à 12 mm.

25. Procédé selon une des revendications 23 ou 24, dans lequel le diamètre de la roue est dans la plage de 6 mm à 10 mm.

26. Procédé selon une quelconque des revendications 1 à 25, dans lequel l'épaisseur de la roue est dans la plage de 1 mm à 3 mm.

27. Roue de comptage (101 ;...701) qui peut être obtenue par le procédé selon une quelconque des revendications 1 à 26.

28. Compteur de doses utilisable dans un inhalateur de doses mesurées, comprenant une roue de comptage (101 ;... 701) selon la revendication 27, dans un mécanisme de comptage de doses.

29. Récipient d'aérosol utilisable dans un inhalateur de doses mesurées, comprenant en outre un compteur de doses selon la revendication 28, attaché à l'inhalateur.

30. Inhalateur de doses mesurées, comprenant un boîtier d'actionneur et un récipient d'aérosol selon la revendication 29 qui peut être reçu dans le boîtier.

31. Kit de pièces comprenant un boîtier d'actionneur et un récipient d'aérosol selon la revendication 29.
